# EUROPEAN PATENT SPECIFICATION

(11) **EP 3 727 356 B1**
(45) Date of publication and mention of the grant of the patent: **23.07.2025**
(21) Application number: 18815759.8
(22) Date of filing: 17.12.2018
(51) Int. Cl.: A61K 31/04, A23K 20/105, A23K 20/111, A23K 20/132, A23K 20/10, A23K 20/20, A23K 20/22, A23K 20/24, A23K 20/26, A23K 20/28, A23K 50/10, A23K 30/00

(54) **STORAGE STABLE MIXTURES**
LAGERBESTÄNDIGE MISCHUNGEN
MÉLANGES STABLES AU STOCKAGE

(30) Priority: 18.12.2017 EP 17207907
(43) Date of publication of application: 28.10.2020
(73) Proprietor: DSM IP Assets B.V., 6221 BE Maastricht (NL)
(72) Inventor: BRUNNER, Dominik Josef, 4303 Kaiseraugst (CH); CLASADONTE, Laure, 4303 Kaiseraugst (CH); GADIENT, Martin Reto, 4303 Kaiseraugst (CH); SCHUEPFER, Roland, 4303 Kaiseraugst (CH)
(74) Representative: dsm-firmenich IP
(86) International application number: PCT/EP2018/085181
(87) International publication number: WO 2019/121502

(56) References cited:
- JESSIE GUYADER ET AL: "Redirection of Metabolic Hydrogen by Inhibiting Methanogenesis in the Rumen Simulation Technique (RUSITEC)", FRONTIERS IN MICROBIOLOGY, vol. 8, 14 March 2017 (2017-03-14), XP055447683, DOI: 10.3389/fmicb.2017.00393
- J. HAISAN ET AL: "The effects of feeding 3-nitrooxypropanol on methane emissions and productivity of Holstein cows in mid lactation", JOURNAL OF DAIRY SCIENCE., vol. 97, no. 5, 1 May 2014 (2014-05-01), US, pages 3110 - 3119, XP055308189, ISSN: 0022-0302, DOI: 10.3168/jds.2013-7834

## Description

The present invention relates to storage stable mixtures comprising propandiol mononitrate and derivatives thereof as well as to the production and use of such forms.

The temperature of the air surrounding the earth is increasing, a process referred to as global warming. One of the main focuses to reduce this warming effect is to reduce the amount of greenhouse gases emitted into the atmosphere. Greenhouse gases are emitted from several different sources, both natural and artificial; however, the two sources with the most emphasis are the agricultural and fossil fuel industries. Within agriculture, ruminants and in particular cattle are the major contributors to the biogenic methane formation, and it has been estimated that the prevention of methane formation from ruminants would almost stabilize atmospheric methane concentrations.

1,3-Propandiol mononitrate (in the following referred to propandiol mononitrate, respectively PDMN) and derivatives thereof have been reported to be highly efficient in reducing the formation of methane in ruminants without affecting microbial fermentation in a way that would be detrimental to the animal (WO2012/084629).

The article by Guyader et al. (J. Guyader, E. M. Ungerfeld and K. Beauchemin, Redirection of Metabolic Hydrogen by Inhibiting Methanogenesis in the Rumen Simulation Technique (RUSITEC), FRONTIERS IN MICROBIOLOGY, vol. 8, 14 March 2017, DOI: 10.3389/fmicb.2017.00393) relates to the inhibition of methanogenesis in the rumen by known methanogenesis inhibitors such as 3-nitrooxypropanol (NOP). In this study, *in vitro* rumen simulation tests were carried out with a substrate composition wherein 0.05% of the active NOP compound is mixed together with 2.81% of dicalcium phosphate and 0.38% of a NOP-carrier which is a mixture of 60% silica and 40% propylene glycol.

However, propandiol mononitrate and derivatives thereof have been found not to be effectively retained under conventional storage conditions, in particular when absorbed onto standard carrier systems commonly used in the feed industry. Furthermore, it has been found that the incorporation of propandiol mononitrate absorbed on silica into standard feed premixes even further reduces the storage stability thereof. The lack of retention of the active in conventional product forms for the feed industry is, however, highly unwanted as accordingly an appropriate dosage is not possible without undue burden, i.e. sophisticated packaging, analysis of the active content before use or overdosing. Moreover, all these methods add significant additional costs-in-use which are not readily accepted by the end user.

Thus, there is an ongoing need for product forms and methods, which overcome the above-mentioned problems by enabling the storage over a period of time without significant losses of the active, i.e. of propandiol mononitrate respectively derivatives thereof.

Surprisingly, it has now been found that the addition of an inorganic phosphate to a powderous formulation of propandiol mononitrate absorbed onto silica, also after incorporation thereof into a mineral premix significantly increased the retention of propandiol mononitrate.

The invention is set out in the appended set of claims 1-13.

Thus, in a first embodiment the present invention relates to a storage stable mixture (1) comprising
(a1) at least 5 wt.-%, based on the total weight of the mixture, of
   a powderous formulation (A) comprising
      (i) a compound of formula (I) 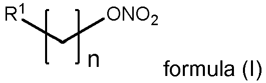 wherein
         n is an integer from 1 to 15
         R¹ is selected from the group consisting of H, C₁-C₆alkyl, phenyl, -OH, -NH₂, -CN, -COOH, -O(C=O)R⁸, -NHC(=O)R⁸, SO₂NHR⁸, and -ONO₂, and
         R⁸ is C₁-C₆alkyl, phenyl, pyridyl such as preferably 2-pyridyl with the proviso that when n is > 3 the hydrocarbon chain may be interrupted by -O- or -NH-, and
      (ii) silica, and
(a2) at least 10 wt.-%, based on the total weight of the mixture, of at least one inorganic phosphate, characterized in that the weight ratio of the at least one inorganic phosphate to the compound of formula (I) is at least 50.

The amount of the powderous formulation (A) in the storage stable mixture (1) is at least 5 wt.-%, more preferably at least 10 wt.-%, based on the total weight of the storage stable mixture (1).

More preferably, the amount of the powderous formulation (A) in the storage stable mixture (1) is selected in the range from 5 to 85 wt.-%, most preferably in the range from 5 to 60 wt.-%, such as in the range of 5 to 50 wt.-%, based on the total weight of the storage stable mixture (1).

The amount of the at least one inorganic phosphate (total) in the storage stable mixture (1) is at least 10 wt.-%, more preferably at least 20 wt.-%, most preferably at least 50 wt.-%, based on the total weight of the storage stable mixture (1).

More preferably, the amount of the at least one inorganic phosphate in the storage stable mixture (1) is selected in the range from 15 to 95 wt.-%, most preferably in the range from 40 to 95 wt.-%, such as in the range of 50 to 95 wt.-%, based on the total weight of the storage stable mixture (1).

The term 'storage-stable' as used herein refers to an improved retention of the compound of formula (I) in the mixture according to the present invention compared to the respective mixture without the at least one inorganic phosphate. Preferably, the retention is improved by at least 10 %, more preferably by at least 20 %, most preferably by at least 30% compared to the respective mixture without any inorganic phosphate.

In another preferred embodiment of the present invention, the weight-ratio (w/w) of the inorganic phosphate to the powderous formulation is at least 5, more preferably at least 10, most preferably at least 15.

In all embodiments of the present invention, the weight-ratio (w/w) of the inorganic phosphate to the compound of formula (I) is at least 50.

Even more advantageously in all embodiments of the present invention the weight-ratio (w/w) of the inorganic phosphate to the compound of formula (I) is selected in the range of 100:1 to 50:1 (i.e. 100 parts of inorganic phosphate to 1 part of a compound of formula (I) to 50 parts of inorganic phosphate to 1 part of a compound of formula (I)), more preferably in the range of 100:1 to 75:1.

The term' powderous formulation' as used herein refers to solid formulations in powder form which freely flow (i.e. free flowing powders).

The amount of silica in the powderous formulations according to the present invention is generally selected in the range of 25 to 90 wt.-%, such as in the range of 30 to 90 wt.-%, 35 to 90 wt.-% or 40 to 90 wt.-%.

In all embodiments of the present invention the powderous formulation (A) is preferably a powderous formulation (B) comprising
(i) at least 0.1 wt-%, based on the total weight of the powderous formulation, of a compound of formula (I), and
(ii) at least 25 wt-%, based on the total weight of the powderous formulation, of silica, and
(iii) 0 to 40 wt-%, based on the total weight of the powderous formulation, of an edible oil.

The term 'edible oil' refers to oils commonly used in feed applications. Preferred edible oils to be used in the powderous formulations are propyleneglycol, canola oil, corn oil, rapeseed oil, sunflower oil, middle chain triglyceride (MCT), soy bean oil and glycerol as well as mixtures thereof. The most preferred edible oil to be used in the powderous formulation is propyleneglycol.

The powderous formulations may furthermore contain small amounts of customary additives commonly used in the preparation of powderous formulations for feed application.

Therefore, the powderous formulations (B) may be powderous formulations (C) which further comprise (iv) 0 to 10 wt-%, based on the total weight of the formulation, of an additive.

The powderous formulation is generally prepared by a process wherein the compound of formula (I) is, optionally diluted in the edible oil and further optionally admixed with the additive(s), sprayed onto or admixed with a silica.

Alternatively, the powderous formulations can be prepared by a process wherein the compound of formula (I) is, optionally in the presence of the edible oil and further optionally admixed with the additive(s), diluted in an organic solvent suitable for the preparation of feed products such as e.g. dichloromethane which dilution is then sprayed onto or admixed with silica followed by evaporation of the organic solvent.

The powderous formulations may be adsorbates.

For the purposes of the present invention, adsorbates are, in particular, preparations in which at least 10 wt.-%, in particular at least 20 wt.-%, preferably at least 30 wt.-%, particularly preferably at least 40 wt.-%, in particular at least 50 wt.-% of the components to be adsorbed (i.e. all constituents of the adsorbate without the silica, i.e. the compound(s) of formula (I), and optionally the edible oil and the additives) are present in the internal pore volume of the silica. The internal pore volume of a carrier can be determined as void volume by the DPB (dibutyl phthalate) method DIN 53601.

Particular preference is given to adsorbates of which at least 60 wt.-%, preferably at least 70 wt.-%, in particular at least 80 wt.-%, is present in the internal pore volume of the silica.

Silica is a well-known carrier material in the feed and food industry and refers to white microspheres of amorphous silica (also referred to as silicone dioxide) and is available in a great variety of particle sizes. Particular suitable silica to be used in powderous formulations is amorphous precipitated silica e.g. available as Ibersil D-250 at IQE Group, Sipernat 2200 at Evonik or Tixosil 68 at Solvay, Zeofree 5170 from J.M. Huber Cooperation or Newsil C50 from Quechen Silicon Chemical Co Ltd.

Preferably the silica which is used in powderous formulations has an average particle size D(v, 0.5) > 200 µm. More preferably the particle size of the silica is selected in the range of 200 µm to 400 µm, most preferably in the range of 250 µm to 380 µm, even more preferably in the range of 300 to 360 µm.

The particle sizes as given herein are measured by a Malvern Master Sizer 2000 following the recommendations outlined in ISO13320-1 for particle size analysis via laser diffraction methods (laser diffraction light scattering). During this laser diffraction measurement, particles are passed through a focused laser beam. The particles scatter light at an angle that is inversely proportional to their size. The angular intensity of the scattered light is then measured by a series of photosensitive detectors. The map of scattering intensity versus angle is the primary source of information used to calculate the particle size. For the measurement of the silica a dry powder feeder (Malvern Scirocco) was used.

Advantageously, the silica which is used in the powderous formulations furthermore exhibits a pH in the range of pH 6 to 8.5 (measured as a 1% suspension in distillated water), such as preferably in the range of pH 7 to 8.

The term 'additive' as used herein refers to additives commonly used in the preparation of powderous formulations for feed application. Preferred additives to be used in the powderous formulations are thickeners, such as in particular gums or cellulose derivatives such as xanthan gum, karaya gum and/ or ethylcellulose.

Particular advantageous powderous formulations are powderous formulation (A) which are powderous formulations (D) consisting essentially of
(i) 1 to 25 wt-%, based on the total weight of the powderous formulation, of a compound of formula (I), and
(ii) at least 20 wt-%, based on the total weight of the powderous formulation, of silica, and
(iii) 5 to 45 wt-%, based on the total weight of the formulation, of at least one edible oil, and
(iv) 0 to 10 wt-%, based on the total weight of the powderous formulation, of an additive.

Even more advantageous powderous formulations are powderous formulations (A) which are powderous formulations (E) consisting essentially of
(i) 2 to 20 wt-%, based on the total weight of the powderous formulation, of a compound of formula (I), and
(ii) at least 25 wt-%, based on the total weight of the powderous formulation, of silica, and
(iii) 10 to 45 wt-%, based on the total weight of the powderous formulation, of an edible oil, and
(iv) 0 to 10 wt-%, based on the total weight of the powderous formulation, of an additive.

An especially preferred powderous formulation is a powderous formulation (A) which is a powderous formulations (F) consisting essentially of
(i) 2 to 15 wt-%, based on the total weight of the powderous formulation, of a compound of formula (I), and
(ii) at least 40 wt-%, based on the total weight of the powderous formulation, of silica, and
(iii) 20 to 40 wt-%, based on the total weight of the powderous formulation, of an edible oil, and
(iv) 0 to 5 wt-%, based on the total weight of the powderous formulation, of an additive.

The compounds of formula (I) preferably have a boiling point below 250°C at 1 bar (760 Torr), preferably a boiling point in the range of 100 and 200°C at 1 bar (760 Torr).

The compounds of formula (I) are known and either commercially available or can be prepared in analogy to the processes as e.g. disclosed in WO2012/084629.

Particular advantageous compounds of formula (I) to be used in the powderous formulations according to the present invention are the compounds wherein n is an integer between 3 and 9 and R¹ is -OH, -COOH or -ONO₂ and with the proviso that if n is 4 the hydrocarbon chain may be interrupted by -NH- such as in particular the compounds of formula R¹-(CH₂)₂-NH-(CH₂)₂-ONO₂. Even more preferred are compounds of formula (I) wherein n is an integer between 3 and 9 and R¹ is -OH, -COOH or -ONO₂.

Even more advantageous compounds of formula (I) to be used in the powderous formulations are propandiol mononitrate (CAS-No: 100502-66-7), 9-nitrooxynonanol, 5-nitroxy pentanoic acid (CAS 74754-56-6), 6-nitroxy hexanoic acid (CAS 74754-55-5), bis(2-hydroxyethyl)amine dinitrate (CAS 20830-49-3), 1,4-bis-nitrooxybutane (CAS 3457-91-8) and 1,5-bis-nitrooxypentane (CAS 3457-92-9). The most preferred compound of formula (I) to be used in the powderous formulations is propandiol mononitrate.

Thus, a very specific powderous formulation is a powderous formulation (A) which is a powderous formulation (G) consisting essentially of
(i) 2 to 15 wt-%, based on the total weight of the powderous formulation, of propandiol mononitrate, and
(ii) at least 45 wt-%, based on the total weight of the powderous formulation, of silica, and
(iii) 20 to 40 wt-%, based on the total weight of the powderous formulation, of propyleneglycol.

The term 'powderous formulation consisting essentially of' indicates that the addition of all wt-% of the listed ingredients of the powderous formulations adds up to 100 wt.-% (i.e. the amount of silica is adjusted accordingly) with the proviso, however, that it cannot be excluded that small amount of impurities or water (moisture) may be present in the powderous formulations such as e.g. in amounts of less than 7 wt.-%, preferably less than 5 wt.-%, more preferably less than 3 wt.-%, which impurities/ water (moisture) are introduced via the respective raw materials or processes used and which are not added separately.

The powderous formulations (A) to (G) with all the preferences and definitions as given herein can additionally be coated with customary coatings in the art such as wax or fats. If present, such coating is generally applied in amounts of 5 to 50 wt.-% based on the total weight of the powderous form. Advantageously, the coating comprises at least one wax and/or at least one fat, which has a dropping point (Tropfpunkt) of from 30 to 85 °C.

The dropping point of a material as used herein refers to the temperature (in °C) when the material begins to melt under standardized conditions. Thus, the material is heated so long until it changes the state of matter from solid to liquid. The dropping point is the temperature when the first dropping is released from the material. The determination of the dropping point is carried out as described in the standard norm DIN ISO 2176.

Particularly suitable waxes to be used as coating include organic compounds consisting of long alkyl chains, natural waxes (plant, animal) which are typically esters of fatty acids and long chain alcohols as well as synthetic waxes, which are long-chain hydrocarbons lacking functional groups.

Particularly suitable fats to be used as coating include a wide group of compounds which are soluble in organic solvents and largely insoluble in water such as hydrogenated fats (or saturated fats) which are generally triesters of glycerol and fatty acids. Suitable fats can have natural or synthetic origin. It is possible to hydrogenate a (poly)unsaturated fat to obtain a hydrogenated (saturated) fat.

Preferred examples of waxes and fats to be used as coating are glycerine monostearate, carnauba wax, candelilla wax, sugarcane wax, palmitic acid, stearic acid hydrogenated cottonseed oil, hydrogenated palm oil and hydrogenated rapeseed oil as well as mixtures thereof.

In a preferred embodiment, the powderous formulations (A) to (G) with all the preferences and definitions as given herein are not coated.

The term 'inorganic phosphate' according to the present invention refers to any inorganic phosphate suitable for feed application (inorganic feed phosphate) such as the calcium phosphates in different forms (mono-, di- and monodi-calcium phosphates) as well as magnesium, calcium-sodium, calcium-magnesium, ammonium and sodium phosphates as well as mixtures thereof.

Generally, the inorganic feed phosphates are derived from natural rock phosphate - the rock being chemically treated to make the phosphorus available for animals in the form of quality feed phosphates.

Preferably in all embodiments of the present invention the at least one inorganic phosphate is a mono-, di- or tri-calcium phosphate such as most preferably dicalcium phosphate.

Commercially available inorganic phosphate grades suitable for the purpose of the present invention are e.g. commercially available at Foodchem International Corporation.

It is well understood, that the storage stable mixtures (1) according to the present invention may contain additional active and/ or feed ingredients and/ or edible oils conventionally used in the feed industry and/ or in feed products.

Thus, in a further embodiment, the present invention relates to a storage stable mixture (1) with all the definitions and preferences as given herein which is a storage stable mixture (2) further comprising (a3) at least one active ingredient and/ or at least one feed ingredient and optionally (a4) at least one edible oil.

In a particular advantageous embodiment, the storage stable mixture (1) is a storage stable mixture (3) comprising
(a1) a powderous formulation (A), (B), (C), (D), (E), (F) or (G), and
(a2) at least one inorganic phosphate, and
(a3) at least one active ingredient and/ or at least one feed ingredient, and optionally
(a4) at least one edible oil.

In a particular preferred embodiment, the at least one active ingredient is selected from the group consisting of water-soluble and/ or fat-soluble vitamins, trace and/ or macro minerals, amino acids as well as mixtures thereof.

Particularly suitable fat-soluble vitamins encompass vitamin A, vitamin D3, vitamin E, and vitamin K, e.g. vitamin K3. Particularly suitable water-soluble vitamins encompass vitamin B12, biotin and choline, vitamin B1, vitamin B2, vitamin B6, niacin, folic acid and panthothenate, e.g. Ca-D-panthothenate as well as mixtures thereof.

Particularly suitable trace minerals encompass manganese (e.g. in the form of manganese oxide), zinc (e.g. in the form of zinc oxide), iron (e.g. in the form of iron sulphate), copper (e.g. in the form of copper sulphate), iodine (e.g. in the form of sodium iodine), selenium, and cobalt as well as mixtures thereof.

Particularly suitable macro minerals encompass calcium (e.g. in the form of limestone and calcium (mono, di or triphosphate), magnesium, phosphorus and sodium (e.g. in the form of sodium chloride) as well as mixtures thereof.

The at least one feed ingredient is selected from the group consisting of roughage and concentrates as well as mixtures thereof.

In one particular advantageous embodiment according to the present invention, the storage stable mixture (1) according to the present invention is a premix (1A) consisting essentially of the ingredients (a1) and (a2), characterized in that the weight ratio of the at least one inorganic phosphate to the compound of formula (I) is at least 50.

In a further preferred embodiment, the storage stable mixture (1) is a premix (1B) consisting essentially of
(a1) at least 5 wt.-%, preferably from 5 to 85 wt.-%, most preferably from 5 to 60 wt.-%, based on the total weight of the premix, of a powderous formulation (A), (B), (C), (D), (E), (F) or (G), and
(a2) at least 10 wt.-%, preferably from of 15 to 95 wt.-%, most preferably from 40 to 95 wt.-%, based on the total weight of the premix, of at least one inorganic phosphate, characterized in that the weight ratio of the at least one inorganic phosphate to the compound of formula (I) is at least 50.

In another preferred embodiment, the storage stable mixture (2) according to the present invention is a premix (2A) consisting essentially of (a1) to (a3) and optionally (a4) and wherein (a3) is at least one additional active ingredient selected from the group consisting of water-soluble and/ or fat-soluble vitamins, trace and/ or macro minerals, amino acids as well as mixtures thereof with the proviso that the ingredients (a1) to (a4) sum up to 100 wt.-%, characterized in that the weight ratio of the at least one inorganic phosphate to the compound of formula (I) is at least 50.

In a further preferred embodiment, the storage stable mixture (2) is a premix (2B) consisting essentially of
(a1) at least 5 wt.-%, preferably from 5 to 20 wt.-%, most preferably from 10 to 15 wt.-%, based on the total weight of the premix, of a powderous formulation (A), (B), (C), (D), (E), (F) or (G), and
(a2) at least 10 wt.-%, preferably from of 15 to 70 wt.-%, most preferably from 20 to 50 wt.-%, based on the total weight of the premix, of at least one inorganic phosphate, and
(a3) at least 5 wt.-%, preferably, from 20 to 80 wt.-%, most preferably from 40 to 70 wt-%, based on the total weight of the premix, of at least one active ingredient selected from the group of water-soluble and/ or fat-soluble vitamins, trace and/ or macro minerals, amino acids as well as mixtures thereof, and
(a4) 0 to 15 wt.-%, preferably 0 to 10 wt.-%, most preferably 0 to 5 wt.-%, based on the total weight of the premix, of at least one edible oil, characterized in that the weight ratio of the at least one inorganic phosphate to the compound of formula (I) is at least 50.

It is noted that next to all the preferences given herein, particular preferred edible oils to be used in the storage stable mixtures according to the present invention are corn oil, rapeseed oil, sunflower oil, canola oil and or soy bean oil as well as mixtures thereof, such as most preferably soy bean oil.

The term 'premix' as used herein designates a preferably uniform mixture of the listed ingredients which are generally used to facilitate uniform dispersion of active ingredients into a larger mix.

The term 'premix consisting essentially of' indicates that the addition of all wt-% of the listed ingredients of the premix adds up to 100 wt.-% with the proviso, however, that it cannot be excluded that small amount of impurities or water (moisture) may be present in the powderous formulations according to the present invention such as e.g. in amounts of less than 7 wt.-%, preferably less than 5 wt.-%, more preferably less than 3 wt.-%, which impurities/ water (moisture) are introduced via the respective raw materials or processes used and which are not added separately.

All the above disclosed premixes can be used as such or admixed to feed products.

Additionally, all the above disclosed premixes can be used in the production of feed products.

It is well understood, that the storage stable mixture (1) according to the present invention may also be a feed product.

Thus, in another preferred embodiment, the storage stable mixture (2) according to the present invention is a feed product (2a) consisting essentially of (a1) to (a3) and optionally (a4) and wherein (a3) is (a3/1) at least one additional active ingredient selected from the group consisting of water-soluble and/ or fat-soluble vitamins, trace and/ or macro minerals, amino acids as well as mixtures thereof and (a3/2) at least one feed ingredient selected from the group of roughage and concentrate, characterized in that the weight ratio of the at least one inorganic phosphate to the compound of formula (I) is at least 50.

The term roughage (also known as forage) and concentrate are well known to a person skilled in the art. Roughage is primarily composed of cellulosic materials such as plant stems and leaves, e.g. hay, introduced grass, native grass, green roughage, straw, tree leaves, etc.; brans such as rice bran, etc. as well as crude fibers such as e.g. brewery's byproducts. The concentrates are generally comprised of the conventional components such as mainly proteins, starch and fats. The concentrate components thus include, for example cereals such as corn, wheat, barley, rye, oat, wheat flour etc.; oil meals such as soybean meal, sunflower oil meal, etc.; feeds of animal origin such as fish meal, mead-and-bone meal, animal oils (e.g. beef oil, lard oil, bone oil, etc.) without being limited thereto.

In another embodiment, the invention relates to the use of an inorganic phosphate to enhance the retention (i.e. reduce the evaporation) of a compound of formula (I) in a powderous formulation comprising (i) the compound according to formula (I) and (ii) silica, characterized in that the weight ratio of the at least one inorganic phosphate to the compound of formula (I) is at least 50.

Preferably, the retention is at least 70 %, preferably at least 80 %, more preferably at least 85%, most preferably at least 90 % such as in particular at least 95 %.

Also disclosed herein but not forming part of the present invention is a method of improving the retention (i.e. reducing the evaporation) of a compound of formula (I) in a powderous formulation, said method comprising admixing the powderous formulation with an inorganic phosphate. The ratio (w/w) of the at least one inorganic phosphate (total) to the powderous formulation may be selected in the range of 50:1 to 1:5, preferably in the range of 40:1 to 1:2, most preferably in the range of 30:1 to 1:1 or 20:1 to 1:1, as these formulations are particular suitable to effectively retain the compound of formula (I) over storage.

Also disclosed herein but not forming part of the present invention is a method of improving the retention (i.e. reducing the evaporation) of a compound of formula (I) in a powderous formulation such as in particular in the powderous formulations (A), (B), (C), (D), (E), (F) or (G), said method comprising the step of preparing a mixture or a premix with all the definitions and preferences as given herein.

Preferably, the mixture/ premix exhibits a retention of at least 80 %, preferably at least 85 % most preferably at least 90 % such as in particular at least 95 %.

The term 'retention' as used therein refers to a retention of the compound of formula (I) with all the definitions and preferences as given herein over a storage time of at least 4 weeks (reclosed (i.e. a bag which has been rolled twice for closure and then fixated with a clip) PE or aluminium bag; 25°C; 50 % relative humidity (r.H.)).

Also disclosed herein but not forming part of the present invention is a method to enhance the retention of a compound of formula (I) in a powderous formulation and with all the definitions and preferences as given herein in a feed product, said method comprising the step of adding a mixture with all the definitions and preferences as given herein with/ to the feed product.

Also disclosed herein but not forming part of the present invention is a method to enhance the storage stability of a feed product comprising a powderous formulation such as in particular the powderous formulations (A), (B), (C), (D), (E), (F) or (G), said method comprising the step of adding at least one inorganic phosphate to the feed composition. Preferably, the ratio (w/w) of the at least one inorganic phosphate (total) to the powderous formulation is selected in the range of 50:1 to 1:5, preferably in the range of 40:1 to 1:2, most preferably in the range of 30:1 to 1:1 or 20:1 to 1:1, as these formulations are particular suitable to effectively retain the compound of formula (I) during storage.

Preferably, the amount of the mixture according to the present invention in the feed product is selected such, that the amount of the compound of formula (I) is in the range of 0.01 to 50g/ kg of feed product, preferably in the range of 0.02 to 25g/ kg of feed product, most preferably in the range of 1 to 10g/ kg of feed product.

The term feed product refers in particular to ruminant feed compositions as well as to feed additives.

It is well understood that all the definitions and preferences of the inorganic phosphate, powderous formulations, compounds of formula (I), active and/ or feed ingredients and edible oils etc. as given herein also apply to the mixtures, premixes, feed products, uses and methods according to the present disclosure as outlined above.

The invention is illustrated by the following Examples. All temperatures are given in °C and all parts and percentages are related to weight.

### Examples

### General information

### A.) HPLC method

Agilent High Performance Liquid Chromatography 1260 Infinity system, using an Aquasil C18, 150 x 3mm, 3µm column and detecting at 210 nm. The column oven was set to 23°C, the autosampler not temperature controlled. The mobile phase consisted of mobile phase A (940mL Milli-Q-water + 60ml acetonitrile + 1mL methane sulfonic acid) and mobile phase B (800ml Milli-Q-water + 200ml acetonitrile + 1mL methane sulfonic acid) which were used in gradient mode (0 min: 0 % B, 15 min: 0 % B, 15.5 min: 100 % B, 21 min: 100 % B, 21.5 min: 0 % B, 25 min: 0 % B (= end of run)) with a flow of 0.4 ml/min.

### B.) Powderous formulation comprising propandiol mononitrate (PF-PDMN)

To 80 g of silica (Newsil C50) placed on a beaker, are added 80 g of a 20 wt.-% propandiol mononitrate (PDMN) solution in propyleneglycol under gentle agitation at room temperature. After 5 minutes agitation, the adsorption is completed and a free-flowing powder is obtained.

### Example 1: Retention of PDMN in PF-PDMN admixed with different inorganic carriers

10 g of PF-PDMN and 90 g of an inorganic carrier as outlined in table 1 have been mixed with a TURBULA^{®} Shaker-Mixer (64 rotations/min) for 10 min, sieved through a 2mm sieve and mixed again for 10 min to obtain homogenous mixtures (100g batches). Then 10 g of the respective mixtures were stored in reclosed PE bags at 25°C under controlled atmosphere (50 % r.H) for 1 month. Afterwards the remaining content of PDMN was determined by HPLC. The results (as relative concentration to the initial value set to 100%) are presented Table 1.

**Table 1 Retention of PDMN in PF-PDMN in dependence of various inorganic carriers**

| **#** | **Inorganic carrier** | **Retention [%]** |
|---|---|---|
| Inv 1 | Dicalcium phosphate | 74 |
| Ref 1 | Sodium chloride | 47 |
| Ref 2 | Zeolith | 45 |
| Ref 3 | Diatomaceous earth (Kieselgur) | 31 |

As can be retrieved from table 1, the use of the inorganic phosphate according to the present invention resulted in an improved retention of the active compared to other inorganic carriers commonly used in the feed industry.

## Claims

1. A storage stable mixture comprising
(a1) at least 5 wt.-%, based on the total weight of the mixture, of a powderous formulation comprising
(i) a compound of formula (I) 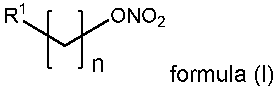 wherein
n is an integer from 1 to 15
R¹ is selected from the group consisting of H, C₁-C₆alkyl, phenyl, -OH, -NH₂, -CN, -COOH, -O(C=O)R⁸, -NHC(=O)R⁸, SO₂NHR⁸, and -ONO₂, and
R⁸ is C₁-C₆alkyl, phenyl, pyridyl such as preferably 2-pyridyl with the proviso that when n is > 3 the hydrocarbon chain may be interrupted by -O- or -NH-, and
(ii) silica, and
(a2) at least 10 wt.-%, based on the total weight of the mixture, of at least one inorganic phosphate, **characterized in that** the weight ratio of the at least one inorganic phosphate to the compound of formula (I) is at least 50.

2. The storage stable mixture according to claim 1, **characterized in that** the compound according to formula (I) is propandiol mononitrate.

3. The storage stable mixture according to claim 1 or 2, **characterized in that** the at least one inorganic phosphate is selected from the group of calcium, magnesium, calcium-sodium, calcium-magnesium, ammonium and sodium phosphates as well as mixtures thereof, preferably from the group of mono-, di- and tricalcium phosphate.

4. The storage stable mixture according to any of the preceding claims, **characterized in that** the weight ratio (w/w) of the inorganic phosphate to the compound of formula 1 is selected in the range of 100:1 to 50:1.

5. The storage stable mixture according to any of the preceding claims, **characterized in that** the powderous formulation consists essentially of
(i) 2 to 20 wt-%, based on the total weight of the powderous formulation, of a compound of formula (I), and
(ii) at least 25 wt-%, based on the total weight of the powderous formulation, of silica, and
(iii) 10 wt-% to 45 wt-%, based on the total weight of the powderous formulation, of an edible oil, and
(iv) 0 to 10 wt-%, based on the total weight of the powderous formulation, of an additive,
wherein the term 'powderous formulation consists essentially of' indicates that the addition of all wt-% of the listed ingredients of the powderous formulations adds up to 100 wt.-%, i.e. the amount of silica is adjusted accordingly,
with the proviso, that small amount of impurities or water (moisture) may be present in the powderous formulations in amounts of less than 7 wt. %, preferably less than 5 wt.-%, more preferably less than 3 wt.-%, which impurities/ water (moisture) are introduced via the respective raw materials and which are not added separately.

6. The storage stable mixture according to claim 5, **characterized in that** the edible oil in the powderous formulation is selected from the group consisting of propyleneglycol, canola oil, corn oil, rapeseed oil, sunflower oil, middle chain triglyceride (MCT) and glycerol as well as mixtures thereof.

7. The storage stable mixture according to claim 5 or 6, **characterized in that** the additive is a thickener selected from the group consisting of gums and/ or cellulose derivatives, preferably from xanthan gum, karaya gum and/ or ethylcellulose.

8. The storage stable mixture according to claims 5 to 7, **characterized in that** the edible oil is propyleneglycol.

9. The storage stable mixture according to any of the preceding claims **characterized in that** the powderous formulation consists essentially of
(i) 2 to 15 wt-%, based on the total weight of the powderous formulation, of propandiol mononitrate, and
(ii) at least 45 wt-%, based on the total weight of the powderous formulation, of silica, and
(iii) 20 to 40 wt-%, based on the total weight of the powderous formulation, of propyleneglycol,
wherein the term 'powderous formulation consists essentially of' indicates that the addition of all wt-% of the listed ingredients of the powderous formulations adds up to 100 wt.-%, i.e. the amount of silica is adjusted accordingly,
with the proviso, that small amount of impurities or water (moisture) may be present in the powderous formulations in amounts of less than 7 wt. %, preferably less than 5 wt.-%, more preferably less than 3 wt.-%, which impurities/ water (moisture) are introduced via the respective raw materials and which are not added separately.

10. The storage stable mixture according to any one of the preceding claims, **characterized in that** the mixture is a premix consisting essentially of (a1) and (a2), wherein the term 'premix consisting essentially of' indicates that the addition of all wt-% of the listed ingredients of the premix adds up to 100 wt.-% with the proviso, that small amount of impurities or water (moisture) may be present in the powderous formulations in amounts of less than 7 wt. %, preferably less than 5 wt.-%, more preferably less than 3 wt.-%, which impurities/ water (moisture) are introduced via the respective raw materials and which are not added separately.

11. The storage stable mixture according to any one of claims 1 to 9, **characterized in that** the mixture is a premix further comprising
(a3) at least one active ingredient selected from the group of water-soluble and/ or fat-soluble vitamins, trace and/ or macro minerals, amino acids as well as mixtures thereof, and optionally
(a4) at least one edible oil,
with the proviso that the amount of ingredients (a1) to (a4) sum up to 100 wt.-%.

12. The storage stable mixture according to any one of claims 1 to 9, **characterized in that** the mixture is a feed product further comprising
(a3/1) at least one active ingredient selected from the group consisting of water-soluble and/ or fat-soluble vitamins, trace and/ or macro minerals, amino acids as well as mixtures thereof and
(a3/2) at least one feed ingredient selected from the group of roughage and concentrates, and optionally
(a4) at least one edible oil.

13. Use of an inorganic phosphate to enhance the retention of a compound of formula (I) 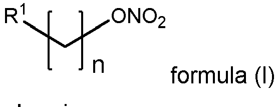 wherein
n is an integer from 1 to 15
R¹ is selected from the group consisting of H, C₁-C₆alkyl, phenyl, -OH, -NH₂, -CN, -COOH, -O(C=O)R⁸, -NHC(=O)R³, SO₂NHR³, and -ONO₂, anc
R⁸ is C₁-C₆alkyl, phenyl, pyridyl such as preferably 2-pyridyl with the proviso that when n is > 3 the hydrocarbon chain may be interrupted by -O- or -NH-in a powderous formulation comprising (i) the compound according to formula (I) and (ii) silica, **characterized in that** the weight ratio of the at least one inorganic phosphate to the compound of formula (I) is at least 50.

## Patentansprüche

1. Lagerbeständige Mischung, umfassend
(a1) mindestens 5 Gew.-%, bezogen auf das Gesamtgewicht der Mischung, einer pulverförmigen Formulierung, umfassend
(i) eine Verbindung der Formel (I) 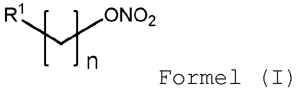 wobei
n eine ganze Zahl von 1 bis 15 ist,
R¹ ausgewählt ist aus der Gruppe bestehend aus H, C¹-C₆-Alkyl, Phenyl, -OH, -NH₂, -CN, -COOH, -O(C=O)R⁸, - NHC(=O)R⁸, SO₂NHR⁸ und -ONO₂ und
R⁸ C₁-C₆-Alkyl, Phenyl, Pyridyl ist, wie vorzugsweise 2-Pyridyl,
mit der Maßgabe, dass bei n > 3 die Kohlenwasserstoffkette durch -O- oder -NH- unterbrochen sein kann, und
(ii) Siliciumdioxid, und
(a2) mindestens 10 Gew.-%, bezogen auf das Gesamtgewicht der Mischung, mindestens eines anorganischen Phosphats, **dadurch gekennzeichnet, dass** das Gewichtsverhältnis des mindestens einen anorganischen Phosphats zu der Verbindung der Formel (I) mindestens 50 beträgt.

2. Lagerbeständige Mischung nach Anspruch 1, **dadurch gekennzeichnet, dass** die Verbindung gemäß Formel (I) Propandiolmononitrat ist.

3. Lagerbeständige Mischung nach Anspruch 1 oder 2, **dadurch gekennzeichnet, dass** das mindestens eine anorganische Phosphat ausgewählt ist aus der Gruppe der Calcium-, Magnesium-, Calciumnatrium-, Calciummagnesium-, Ammonium- und Natriumphosphate sowie Mischungen davon, vorzugsweise aus der Gruppe Mono-, Di- und Tricalciumphosphat.

4. Lagerbeständige Mischung nach einem der vorhergehenden Ansprüche, **dadurch gekennzeichnet, dass** das Gewichtsverhältnis (w/w) des anorganischen Phosphats zu der Verbindung der Formel 1 im Bereich von 100:1 bis 50:1 ausgewählt ist.

5. Lagerbeständige Mischung nach einem der vorhergehenden Ansprüche, **dadurch gekennzeichnet, dass** die pulverförmige Formulierung im Wesentlichen besteht aus
(i) 2 bis 20 Gew.-%, bezogen auf das Gesamtgewicht der pulverförmigen Formulierung, einer Verbindung der Formel (I) und
(ii) mindestens 25 Gew.-%, bezogen auf das Gesamtgewicht der pulverförmigen Formulierung, Siliciumdioxid und
(iii) 10 Gew.-% bis 45 Gew.-%, bezogen auf das Gesamtgewicht der pulverförmigen Formulierung, eines Speiseöls und
(iv) 0 bis 10 Gew.-%, bezogen auf das Gesamtgewicht der pulverförmigen Formulierung, eines Additivs,
wobei der Begriff "pulverförmige Formulierung im Wesentlichen besteht aus" angibt, dass sich die Zugabe aller Gew.-% der aufgelisteten Inhaltsstoffe der pulverförmigen Formulierungen zu 100 Gew.-% addiert, d. h. dass die Menge an Siliciumdioxid entsprechend angepasst wird, mit der Maßgabe, dass eine geringe Menge an Verunreinigungen oder Wasser (Feuchtigkeit) in den pulverförmigen Formulierungen in Mengen von weniger als 7 Gew.-%, vorzugsweise weniger als 5 Gew.-%, bevorzugter weniger als 3 Gew.-% vorhanden sein kann, wobei die Verunreinigungen/das Wasser (Feuchtigkeit) über die jeweiligen Rohstoffe eingebracht und nicht separat zugegeben werden.

6. Lagerbeständige Mischung nach Anspruch 5, **dadurch gekennzeichnet, dass** das Speiseöl in der pulverförmigen Formulierung ausgewählt ist aus der Gruppe bestehend aus Propylenglykol, Canolaöl, Maisöl, Rapsöl, Sonnenblumenöl, mittelkettigem Triglycerid (MCT) und Glycerin sowie Mischungen davon.

7. Lagerbeständige Mischung nach Anspruch 5 oder 6, **dadurch gekennzeichnet, dass** das Additiv ein Verdickungsmittel ausgewählt aus der Gruppe bestehend aus Gummis und/oder Cellulosederivaten, vorzugsweise aus Xanthangummi, Karayagummi und/oder Ethylcellulose, ist.

8. Lagerbeständige Mischung nach den Ansprüchen 5 bis 7, **dadurch gekennzeichnet, dass** das Speiseöl Propylenglykol ist.

9. Lagerbeständige Mischung nach einem der vorhergehenden Ansprüche, **dadurch gekennzeichnet, dass** die pulverförmige Formulierung im Wesentlichen besteht aus
(i) 2 bis 15 Gew.-%, bezogen auf das Gesamtgewicht der pulverförmigen Formulierung, Propandiolmononitrat, und
(ii) mindestens 45 Gew.-%, bezogen auf das Gesamtgewicht der pulverförmigen Formulierung, Siliciumdioxid und
(iii) 20 bis 40 Gew.-%, bezogen auf das Gesamtgewicht der pulverförmigen Formulierung, Propylenglykol,
wobei der Begriff "pulverförmige Formulierung im Wesentlichen besteht aus" angibt, dass sich die Zugabe aller Gew.-% der aufgelisteten Inhaltsstoffe der pulverförmigen Formulierungen zu 100 Gew.-% addiert, d. h. dass die Menge an Siliciumdioxid entsprechend angepasst wird, mit der Maßgabe, dass eine geringe Menge an Verunreinigungen oder Wasser (Feuchtigkeit) in den pulverförmigen Formulierungen in Mengen von weniger als 7 Gew.-%, vorzugsweise weniger als 5 Gew.-%, bevorzugter weniger als 3 Gew.-% vorhanden sein kann, wobei die Verunreinigungen/das Wasser (Feuchtigkeit) über die jeweiligen Rohstoffe eingebracht und nicht separat zugegeben werden.

10. Lagerbeständige Mischung nach einem der vorhergehenden Ansprüche, **dadurch gekennzeichnet, dass** die Mischung eine Vormischung im Wesentlichen bestehend aus (a1) und (a2) ist, wobei der Begriff "Vormischung im Wesentlichen bestehend aus" angibt, dass sich die Zugabe aller Gew.-% der aufgelisteten Inhaltsstoffe der Vormischung zu 100 Gew.-% addiert, mit der Maßgabe, dass eine geringe Menge an Verunreinigungen oder Wasser (Feuchtigkeit) in den pulverförmigen Formulierungen in Mengen von weniger als 7 Gew.-%, vorzugsweise weniger als 5 Gew.-%, bevorzugter weniger als 3 Gew.-% vorhanden sein kann, wobei die Verunreinigungen/das Wasser (Feuchtigkeit) über die jeweiligen Rohstoffe eingebracht und nicht separat zugegeben werden.

11. Lagerbeständige Mischung nach einem der Ansprüche 1 bis 9, **dadurch gekennzeichnet, dass** die Mischung eine Vormischung ist, weiterhin umfassend
(a3) mindestens einen Wirkstoff, ausgewählt aus der Gruppe der wasserlöslichen und/oder fettlöslichen Vitamine, Spurenelemente und/oder Makromineralien, Aminosäuren sowie Mischungen davon, und gegebenenfalls
(a4) mindestens ein Speiseöl,
mit der Maßgabe, dass sich die Menge der Inhaltsstoffe (a1) bis (a4) auf 100 Gew.-% summiert.

12. Lagerbeständige Mischung nach einem der Ansprüche 1 bis 9, **dadurch gekennzeichnet, dass** die Mischung ein Futterprodukt ist, weiterhin umfassend
(a3/1) mindestens einen Wirkstoff, ausgewählt aus der Gruppe bestehend aus wasserlöslichen und/oder fettlöslichen Vitaminen, Spurenelementen und/oder Makromineralien, Aminosäuren sowie Mischungen davon, und
(a3/2) mindestens einen Futtermittelinhaltsstoff, ausgewählt aus der Gruppe der Raufutter und Konzentrate, und gegebenenfalls
(a4) mindestens ein Speiseöl.

13. Verwendung eines anorganischen Phosphats zur Verbesserung der Retention einer Verbindung der Formel (I) 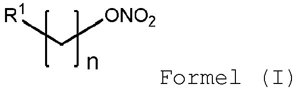 wobei
n eine ganze Zahl von 1 bis 15 ist,
R¹ ausgewählt ist aus der Gruppe bestehend aus H, C₁-C₆-Alkyl, Phenyl, -OH, -NH₂, -CN, -COOH, -O(C=O)R⁸, - NHC (=O)R⁸, SO₂NHR⁸ und -ONO₂ und
R⁸ C₁-C₆-Alkyl, Phenyl, Pyridyl ist, wie vorzugsweise 2-Pyridyl,
mit der Maßgabe, dass bei n > 3 die Kohlenwasserstoffkette durch -O- oder -NH- unterbrochen sein kann, in einer pulverförmigen Formulierung, umfassend (i) die Verbindung gemäß Formel (I) und (ii) Siliciumdioxid, **dadurch gekennzeichnet, dass** das Gewichtsverhältnis des mindestens einen anorganischen Phosphats zu der Verbindung der Formel (I) mindestens 50 beträgt.

## Revendications

1. Mélange stable au stockage comprenant
(a1) au moins 5 % en poids, par rapport au poids total du mélange, d'une formulation pulvérulente comprenant
(i) un composé de formule (I) 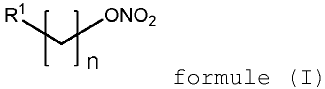 dans laquelle
n est un entier de 1 à 15
R¹ est choisi dans le groupe constitué par H, C₁-C₆alkyle, phényle, -OH, -NH₂, -CN, -COOH, -O(C=O)R⁸, -NHC(=O)R⁸, SO₂NHR⁸ et -ONO₂, et
R⁸ est C₁-C₆alkyle, phényle, pyridinyle tel que de préférence 2-pyridinyle
à condition que lorsque n est > 3, la chaîne hydrocarbonée puisse être interrompue par -O- ou -NH-, et
(ii) une silice, et
(a2) au moins 10 % en poids, par rapport au poids total du mélange, d'au moins un phosphate inorganique, **caractérisé en ce que** le rapport pondéral de l'au moins un phosphate inorganique sur le composé de formule (I) est d'au moins 50.

2. Mélange stable au stockage selon la revendication 1, **caractérisé en ce que** le composé selon la formule (I) est le mononitrate de propanediol.

3. Mélange stable au stockage selon la revendication 1 ou 2, **caractérisé en ce que** l'au moins un phosphate inorganique est choisi dans le groupe constitué par les phosphates de calcium, de magnésium, de calcium-sodium, de calcium-magnésium, d'ammonium et de sodium ainsi que des mélanges correspondants, de préférence dans le groupe constitué par le phosphate mono-, di- et tricalcique.

4. Mélange stable au stockage selon l'une quelconque des revendications précédentes, **caractérisé en ce que** le rapport pondéral (p/p) du phosphate inorganique sur le composé de formule 1 est choisi dans la plage de 100:1 à 50:1.

5. Mélange stable au stockage selon l'une quelconque des revendications précédentes, **caractérisé en ce que** la formulation pulvérulente est constituée essentiellement de
(i) 2 à 20 % en poids, par rapport au poids total de la formulation pulvérulente, d'un composé de formule (I), et
(ii) au moins 25 % en poids, par rapport au poids total de la formulation pulvérulente, de silice, et
(iii) 10 % en poids à 45 % en poids, par rapport au poids total de la formulation pulvérulente, d'une huile comestible, et
(iv) 0 à 10 % en poids, par rapport au poids total de la formulation pulvérulente, d'un additif,
dans lequel le terme « formulation pulvérulente constituée essentiellement de » indique que l'ajout de tous les % en poids des ingrédients listés des formulations pulvérulentes représente jusqu'à 100 % en poids, c'est-à-dire que la quantité de silice est ajustée en conséquence, à condition qu'une petite quantité d'impuretés ou d'eau (humidité) puisse être présente dans les formulations pulvérulentes en des quantités inférieures à 7 % en poids, de préférence inférieures à 5 % en poids, plus préférablement inférieures à 3 % en poids, lesdites impuretés/eau (humidité) étant introduites par l'intermédiaire des matières premières respectives et n'étant pas ajoutées séparément.

6. Mélange stable au stockage selon la revendication 5, **caractérisé en ce que** l'huile comestible dans la formulation pulvérulente est choisie dans le groupe constitué par le propylène glycol, l'huile de canola, l'huile de maïs, l'huile de colza, l'huile de tournesol, un triglycéride à chaîne moyenne (MCT) et le glycérol ainsi que des mélanges correspondants.

7. Mélange stable au stockage selon la revendication 5 ou 6, **caractérisé en ce que** l'additif est un épaississant choisi dans le groupe constitué par les gommes et/ou les dérivés de cellulose, de préférence la gomme xanthane, la gomme karaya et/ou l'éthylcellulose.

8. Mélange stable au stockage selon les revendications 5 à 7, **caractérisé en ce que** l'huile comestible est le propylèneglycol.

9. Mélange stable au stockage selon l'une quelconque des revendications précédentes, **caractérisé en ce que** la formulation pulvérulente est constituée essentiellement de
(i) 2 à 15 % en poids, par rapport au poids total de la formulation pulvérulente, de mononitrate de propanediol, et
(ii) au moins 45 % en poids, par rapport au poids total de la formulation pulvérulente, de silice, et
(iii) 20 à 40 % en poids, par rapport au poids total de la formulation pulvérulente, de propylène glycol,
dans lequel le terme « formulation pulvérulente constituée essentiellement de » indique que l'ajout de tous les % en poids des ingrédients listés des formulations pulvérulentes représente jusqu'à 100 % en poids, c'est-à-dire que la quantité de silice est ajustée en conséquence, à condition qu'une petite quantité d'impuretés ou d'eau (humidité) puisse être présente dans les formulations pulvérulentes en des quantités inférieures à 7 % en poids, de préférence inférieures à 5 % en poids, plus préférablement inférieures à 3 % en poids, lesdites impuretés/eau (humidité) étant introduites par l'intermédiaire des matières premières respectives et n'étant pas ajoutées séparément.

10. Mélange stable au stockage selon l'une quelconque des revendications précédentes, **caractérisé en ce que** le mélange est un prémélange constitué essentiellement de (a1) et (a2), dans lequel le terme « prémélange constitué essentiellement de » indique que l'ajout de tous les % en poids des ingrédients listés du prémélange représente jusqu'à 100 % en poids, à condition qu'une petite quantité d'impuretés ou d'eau (humidité) puisse être présente dans les formulations pulvérulentes en des quantités inférieures à 7 % en poids, de préférence inférieures à 5 % en poids, plus préférablement inférieures à 3 % en poids, lesdites impuretés/eau (humidité) étant introduites par l'intermédiaire des matières premières respectives et n'étant pas ajoutées séparément.

11. Mélange stable au stockage selon l'une quelconque des revendications 1 à 9, **caractérisé en ce que** le mélange est un prémélange comprenant en outre
(a3) au moins un ingrédient actif choisi dans le groupe constitué par des vitamines solubles dans l'eau et/ou liposolubles, des oligo-éléments et/ou macro-minéraux, des acides aminés ainsi que des mélanges correspondants, et éventuellement
(a4) au moins une huile comestible,
à condition que la somme des ingrédients (a1) à (a4) totalise 100 % en poids.

12. Mélange stable au stockage selon l'une quelconque des revendications 1 à 9, **caractérisé en ce que** le mélange est un produit alimentaire comprenant en outre
(a3/1) au moins un ingrédient actif choisi dans le groupe constitué par des vitamines solubles dans l'eau et/ou liposolubles, des oligo-éléments et/ou macro-minéraux, des acides aminés ainsi que des mélanges correspondants et
(a3/2) au moins un ingrédient alimentaire choisi dans le groupe constitué par des fourrages grossiers et des concentrés, et éventuellement
(a4) au moins une huile comestible.

13. Utilisation d'un phosphate inorganique pour améliorer la rétention d'un composé de formule (I) 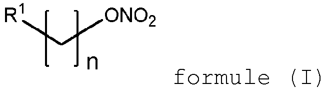 dans laquelle
n est un entier de 1 à 15
R¹ est choisi dans le groupe constitué par H, C₁-C₆alkyle, phényle, -OH, -NH₂, -CN, -COOH, -O(C=O)R⁸, -NHC(=O)R⁸, SO₂NHR⁸ et -ONO₂, et
R⁸ est C₁-C₆alkyle, phényle, pyridinyle tel que de préférence 2-pyridinyle
à condition que, lorsque n est > 3, la chaîne hydrocarbonée puisse être interrompue par -O- ou -NH-dans une formulation pulvérulente comprenant (i) le composé de formule (I) et (ii) une silice, **caractérisée en ce que** le rapport pondéral de l'au moins un phosphate inorganique sur le composé de formule (I) est d'au moins 50.
